# EUROPEAN PATENT SPECIFICATION

(11) **EP 4 212 199 B1**
(45) Date of publication and mention of the grant of the patent: **12.11.2025**
(21) Application number: 21866792.1
(22) Date of filing: 08.09.2021
(51) Int. Cl.: A61M 25/00

(54) **MEDICAL TUBE, AND MEDICAL TUBE POSITION DETECTION SYSTEM**
MEDIZINISCHER SCHLAUCH UND SYSTEM ZUR ERKENNUNG DER POSITION DES MEDIZINISCHEN SCHLAUCHES
TUBE MÉDICAL ET SYSTÈME DE DÉTECTION DE POSITION DE TUBE MÉDICAL

(30) Priority: 09.09.2020 JP 2020151167
(43) Date of publication of application: 19.07.2023
(60) Divisional application: 25207260.8
(73) Proprietor: Otsuka Clinical Solutions, Inc., Uruma, Okinawa 904-2311 (JP); Pax Co., Ltd., Tokyo 104-0054 (JP); Otsuka Pharmaceutical Factory, Inc., Naruto-shi, Tokushima 772-8601 (JP)
(72) Inventor: MIIKE, Shinya, Tokyo 113-0033 (JP); SUZUKI, Yutaka, Tokyo 104-0054 (JP)
(74) Representative: Maiwald GmbH
(86) International application number: PCT/JP2021/033021
(87) International publication number: WO 2022/054833

(56) References cited:
- WO-A1-2021/024992
- US-A1- 2020 205 640

## Description

### [Technical Field]

The present disclosure relates to a medical tube and a medical tube position detection system.

### [Background Art]

By means of a technique called nasogastric tube feeding, a nutrient and/or the like is directly supplied to a stomach of a patient who has difficulties in oral ingestion of a nutrient and/or the like. Specifically, a medical tube is inserted through a nasal cavity of a patient, and a distal end part thereof arrives at a stomach via an esophagus. Then, a nutrient and/or the like is injected from a proximal end part of the medical tube that is positioned outside a body.

Here, there has been known a technique for detecting that the distal end part of the medical tube has arrived at a stomach via an esophagus.

For example, Patent Literature 1 discloses a catheter device including a catheter to be inserted into a body, a light source part including a laser diode to emit visible red light, and a fiber to guide the visible red light emitted from the light source part to the vicinity of a distal end part of the catheter. With this configuration, when the catheter is inserted into a stomach of a patient, the light emitted in the vicinity of the distal end part of the catheter can visually recognized from the outside of the body, thereby being able to detect the position of the distal end part of the catheter from the outside of the body.

US 2020/0205640 A1 relates to an illumination system for illuminating a lumen within a body of a patient.

### [Citation List]

### [Patent Literature]

[PTL 1] Japanese Patent Application Publidation No.2016-087091

### [Summary of Invention]

### [Technical Problem]

However, in the catheter device described in Patent Literature 1, it may be difficult to visually recognize, from the outside of the body, the light emitted from the distal end of the fiber. Specifically, when the distal end of the fiber in a stomach of a patient is not oriented toward the abdominal side of the patient, it is difficult to visually recognize the light from the outside of the body. Further, it is difficult to operate it so as to orient the distal end of the fiber in the stomach toward the abdominal side of the patient.

The present disclosure is directed to provision of such a medical tube that a position of a distal end part thereof is easily detectable from an outside of a body of a patient, regardless of orientation of the distal end part in the body.

### [Solution to Problem]

An aspect of the disclosure for achieving an object described above is a medical tube comprising: a main body part having a tubular shape; at least one opening formed in a side face of a main body part; and a light transmitting part formed at one end of the main body part, the light transmitting part having a hemispherical face shape, the light transmitting part being configured to emit light from a light source. Other features of the present disclosure will become apparent from the description in the present specification and the accompanying drawings described below.

The present invention and the scope thereof is defined by the appended claims. Embodiments not falling under these claims are for reference purposes only.

### [Advantageous Effects of Invention]

According to the present disclosure, it is possible to provide such a medical tube that a position of a distal end part thereof is easily detectable from an outside of a body of a patient, regardless of orientation of the distal end part inserted into the body.

### [Brief Description of Drawings]

Figures 1 to 4 disclose an embodiment not being part of the invention as defined by the claims.
Fig. 1 is a diagram illustrating an overall configuration of a medical tube position detection system according to a first embodiment.
Fig. 2 is a diagram illustrating a configuration of a medical tube in a vicinity of a distal end part thereof according to a first embodiment.
Fig. 3 is a diagram illustrating a configuration of a medical tube in a vicinity of a distal end part thereof according to a first embodiment.
Fig. 4 is a diagram illustrating a configuration of a medical tube in a vicinity of a distal end part thereof according to a first embodiment.
Fig. 5 is a diagram illustrating an overall configuration of a medical tube position detection system according to a second embodiment.
Fig. 6 is a diagram illustrating a configuration of a medical tube in a vicinity of a distal end part thereof according to a second embodiment.
Fig. 7 is a diagram illustrating a configuration of a medical tube in vicinities of a distal end part thereof and a proximal end part according to a second embodiment.
Fig. 8 is a diagram illustrating a configuration of a medical tube in a vicinity of a distal end part thereof according to a second embodiment.
Fig. 9 is a diagram illustrating a configuration of a medical tube in a vicinity of a distal end part thereof in a modification example.
Fig. 10 is a diagram illustrating a configuration of a medical tube in a vicinity of a distal end part thereof in a modification example.
Fig. 11 is a diagram illustrating a configuration of a medical tube in a vicinity of a distal end part thereof in a modification example.
Fig. 12 is a diagram illustrating a configuration of a medical tube in a vicinity of a distal end part thereof in a modification example.
Fig. 13 is a diagram illustrating a configuration of a medical tube in a vicinity of a distal end part thereof in a modification example.

### [Description of Embodiments]

### First Embodiment

### == Medical Tube Position Detection System ==

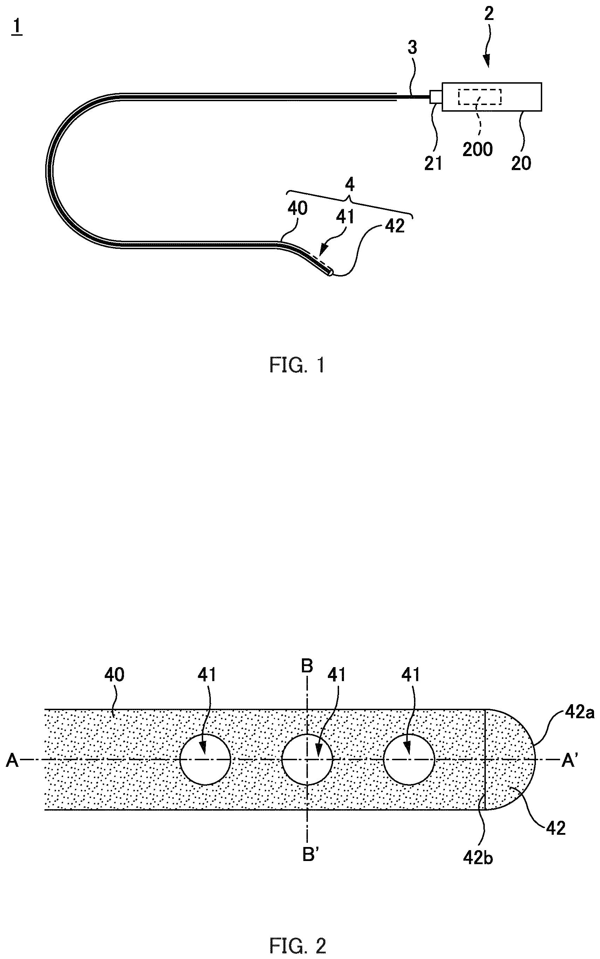

Fig. 1 is a diagram illustrating an overall configuration of a medical tube position detection system 1 according to an embodiment of the present disclosure. The medical tube position detection system 1 includes a light source device 2, a light guide 3, and a medical tube 4.

### [Light Source Device]

The light source device 2 supplies light to the light guide 3. The light source device 2 includes a casing 20 and a light collecting part 21. The casing 20 includes a light source 200 thereinside. The light source 200 generates light including a wavelength passing through a living body. The light collecting part 21 includes a lens group (not illustrated) to collect the light generated by the light source 200, and emits the collected light to the outside. One end part of the light guide 3 is connected to the light collecting part 21, and thus the light source device 2 is capable of supplying the light from the light source 200 to the light guide 3. The light from the light source 200 is used to detect the position of a distal end part of the medical tube 4.

### [Light guide]

The light guide 3 is a flexible member having a long length. The light guide 3 is insertable into the medical tube 4, and guides the light from the light source 200. The light entering the one end part of the light guide 3 propagates inside the light guide 3, and is emitted from the other end part of the light guide 3. In the following description, in the light guide 3, an end part thereof on the side on which the light from the light source 200 is incident is referred to as "proximal end part (of the light guide)", and an end part thereof on the side on which the light is emitted is referred to as "distal end part (of the light guide)".

The light guide 3 is, for example, an optical fiber, and includes a core and a cladding. The core allows the light from the light source 200 to be transmitted therethrough. Examples of a material of the core include quartz, glass, and plastic, and the like. The cladding fills the outside of the core. Examples of a material of the cladding include quartz, glass, plastic, and the like, and the material has a refraction index smaller than that of the core. The light entering the light guide 3 propagates through the core while repeating total reflection at an interface between the core and the cladding.

### [Medical Tube]

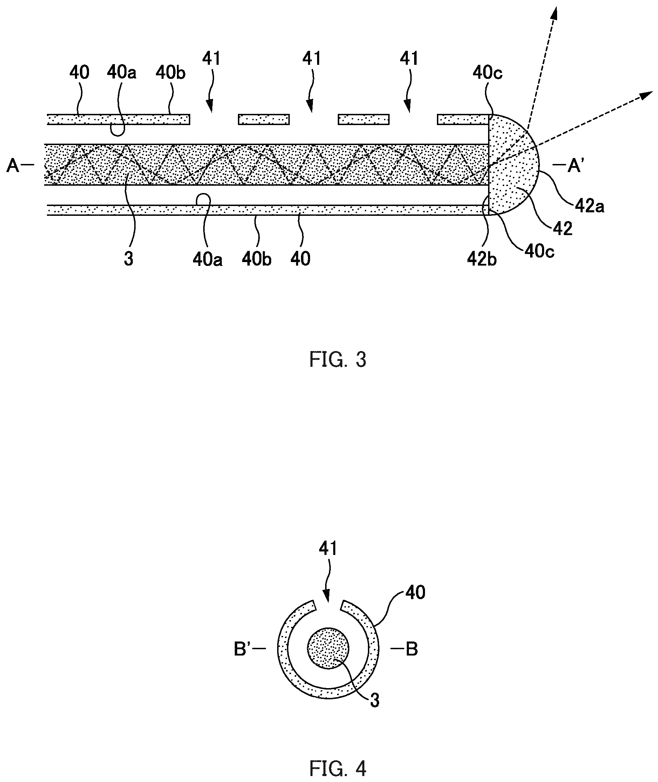

Each of Fig. 2 to Fig. 4 is a diagram illustrating a configuration of the medical tube 4 in the vicinity of the distal end part thereof according to an embodiment of the present disclosure. Fig. 2 is an enlarged diagram illustrating the vicinity of the distal end part of the medical tube 4. Fig. 3 is a cross-sectional view taken along a line A-A' illustrated in Fig. 2. Fig. 4 is a cross-sectional view taken along a line B-B' illustrated in Fig. 2.

The medical tube 4 is a flexible tubular body having a long length. The medical tube 4 is used for a technique of nasogastric tube feeding. In the medical tube 4, an end part thereof on the side on which the tube is inserted into a body of a patient is referred to as "distal end part (of the medical tube)", and the other end part thereof is referred to as "proximal end part (of the medical tube)".

An operator inserts the medical tube 4 through a nasal cavity or a mouth of a patient, and causes the distal end part of the medical tube 4 to arrive at a stomach via an esophagus. Then, a nutrient and/or the like is injected from the proximal end part of the medical tube 4 that is positioned outside a body of the patient. This makes it possible to directly supply a nutrient and/or the like to the stomach of the patient.

As illustrated in Fig. 1, the medical tube 4 includes a main body part 40, an opening 41, and a light transmitting part 42.

The main body part 40 is a tubular member. A hollow part of the main body part 40 communicates from a proximal end part to a distal end part of the main body part 40. The proximal end part of the main body part 40 is an end part on a side from which a nutrient and/or the like is to be injected. The distal end part of the main body part 40 is an end part on a side on which the light transmitting part 42 is formed.

The main body part 40 includes an inner circumferential face 40a, an outer circumferential face 40b, an end face on the proximal end part side (not illustrated), and an end face 40c on the distal end part side (Fig. 3).

The main body part 40 allows the light guide 3 to be inserted into the hollow part thereof, the light guide 3 being configured to transmit the light from the light source 200 to the light transmitting part 42 (Fig. 3, Fig. 4). In other words, the diameter of the hollow part of the main body part 40 is larger than the diameter of the light guide 3.

The main body part 40 is formed of a material having flexibility. Specific examples of a material of the main body part 40 may include silicone, polypropylene, elastomer, a fluorocarbon resin, and the like.

The opening 41 is formed in a side face of the main body part 40. The opening 41 is a hole for introducing, into a stomach of a patient, a nutrient and/or the like injected from the proximal end part of the main body part 40.

Here, as illustrated in Fig. 2 and the like, the opening 41 is formed in the side face of the main body part 40 in the vicinity of the distal end part thereof. The side face in the vicinity of the distal end part is a side face to be positioned in a stomach in a state in which the main body part 40 is inserted into a body of a patient.

In an embodiment of the present disclosure, three openings 41 are formed in the side face of the main body part 40. The three openings 41 have a circular shape, and have the same size. The three openings 41 are arranged in a row in a longitudinal direction of the main body part 40.

Note that the number, the shape, the size, the arrangement, and the like of the openings 41 may not be limited to this aspect, as long as, for example, at least one opening 41 is formed in the side face of the main body part 40 in the vicinity of the distal end part of the main body part 40. Alternatively, a plurality of openings 41 may have different shapes and/or sizes.

The light transmitting part 42 is a member to emit the light from the light source 200. The light transmitting part 42 is formed at one end (distal end part) of the main body part 40. The light transmitting part 42 has a hemispherical face shape. Here, the term "hemispherical face" is not limited to a perfect hemispherical face, but includes a substantially hemispherical face obtained by deforming the perfect hemispherical face. Here, the term "substantially hemispherical face" indicates a shape capable of emitting the light from the light source 200 at an irradiation angle nearly equal to that of the light transmitting part 42 having the perfect hemispherical face.

The light transmitting part 42 is formed of a light transmissive material. Specific examples of a material of the light transmitting part 42 may include silicone, polypropylene, elastomer, a fluorocarbon resin, and the like.

The light transmitting part 42 includes a curved part 42a and a bottom part 42b. The curved part 42a is a hemisphere portion of a face of the light transmitting part 42. The bottom part 42b is a flat portion of a face of the light transmitting part 42. The shape of the bottom part 42b is circular, and the diameter thereof is equal to the diameter of the main body part 40. The bottom part 42b of the light transmitting part 42 is connected to the end face 40c of the main body part 40 on the distal end part side. Note that the main body part 40 and the light transmitting part 42 may be formed in an integrated manner.

### [Propagation of Light]

Next, with reference to Fig. 3, a description is given of propagation of the light from the light source 200 until the light is emitted from the distal end part of the medical tube 4.

When the position of the distal end part of the medical tube 4 is detected, the light guide 3 has been inserted in the medical tube 4 as illustrated in Fig. 3. Note that the medical tube 4 may be inserted into a body of a patient in a state in which the light guide 3 has been inserted in the medical tube **4.** Alternatively, the light guide 3 may be inserted into the medical tube 4 after the medical tube 4 is inserted into a body of a patient.

Fig. 3 illustrates an aspect of usage in a state in which a distal end part of the light guide 3 is in contact with the bottom part 42b of the light transmitting part 42. Note that an embodiment is not limited to this aspect, as long as a configuration is such that the light emitted from the distal end part of the light guide 3 enters the light transmitting part 42 from the bottom part 42b. For example, the bottom part 42b of the light transmitting part 42 and the distal end part of the light guide 3 may have an interval therebetween.

In the state in Fig. 3, first, the light from the light source 200 enters the light guide 3 from the proximal end part of the light guide 3. The light having entered the light guide 3 propagates to the the distal end part side of the light guide 3 while repeating reflections in the light guide 3. The light having arrived at the distal end part of the light guide 3 enters the light transmitting part 42 from the bottom part 42b of the light transmitting part 42 (Fig. 3).

Part of the light having entered the light transmitting part 42 is emitted from the curved part 42a to the outside of the light transmitting part 42. In this case, the traveling direction of the light changes according to the refraction index of the light transmitting part 42 and the angle of incidence at the curved part 42a (Fig. 3). Another part of the light having entered the light transmitting part 42 repeats reflections at the curved part 42a or the bottom part 42b one or more times, and then is emitted from the curved part 42a to the outside of the light transmitting part 42. In this case as well, the traveling direction of the light changes according to the refraction index of the light transmitting part 42 and the angle of incidence at the curved part 42a.

As described above, the light having entered the light transmitting part 42 is refracted at the curved part 42a, and is emitted to various directions.

### Second Embodiment

### == Medical Tube Position Detection System ==

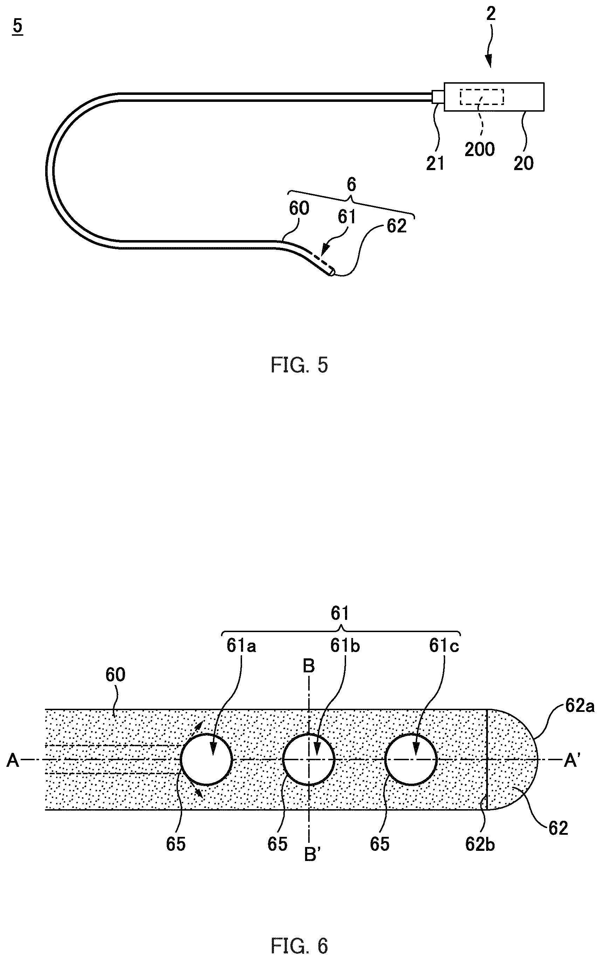

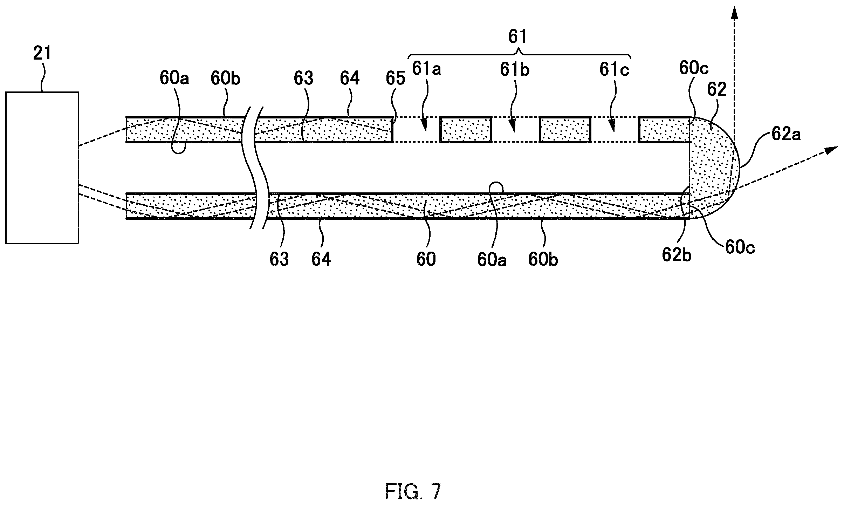

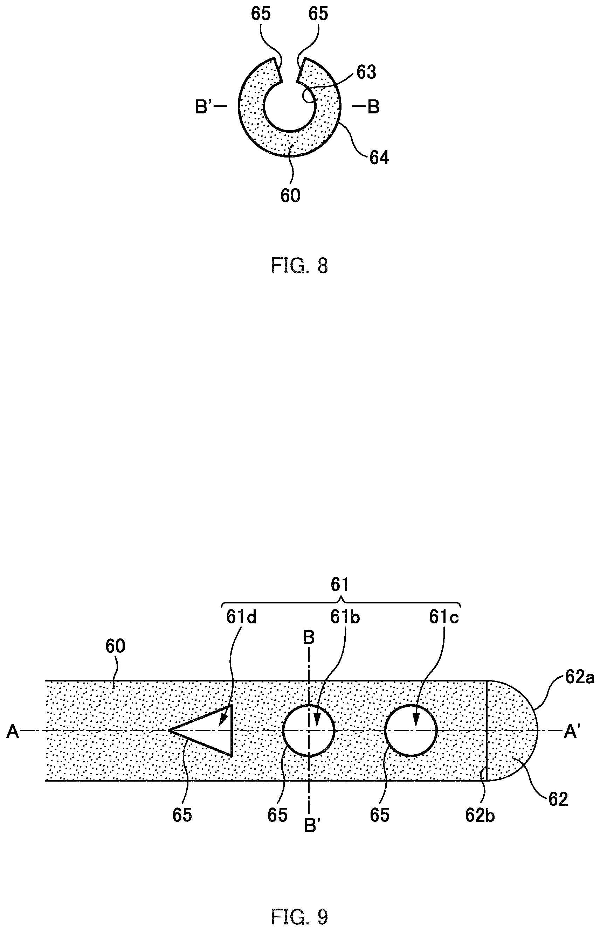

Next, with reference to Fig. 5 to Fig. 8, a medical tube position detection system 5 according to a second embodiment is described. Fig. 5 is a diagram illustrating an overall configuration of the medical tube position detection system 5 according to an embodiment of the present disclosure. Each of Fig. 6 to Fig. 8 is a diagram illustrating a configuration of a medical tube 6 in the vicinity of a distal end part thereof in the medical tube position detection system 5 according to an embodiment of the present disclosure. Fig. 6 is a side view of the medical tube 6 in the vicinity of the distal end part thereof. Fig. 7 is a cross-sectional view taken along a line A-A' illustrated in Fig. 6. Note that Fig. 7 also illustrates the light collecting part 21 and the medical tube 6 in the vicinity of the proximal end part thereof. Fig. 8 is a cross-sectional view taken along a line B-B' illustrated in Fig. 6.

The medical tube position detection system 5 includes the light source device 2 and the medical tube 6.
The medical tube position detection system 5 according to an embodiment of the present disclosure does not include the light guide 3, as compared with the medical tube position detection system 1 according to the first embodiment (Fig. 5, Fig. 7, Fig. 8). The medical tube 6 according to an embodiment of the present disclosure has a configuration different from that of the medical tube 4 according to the first embodiment. The following mainly describes differences between the first embodiment and the second embodiment.

### [Medical Tube]

The medical tube 6 includes a main body part 60, an opening 61, a light transmitting part 62, a first reflection layer 63, a second reflection layer 64, and a third reflection layer 65.

The main body part 60 is different from the main body part 40 in the first embodiment in that the main body part 60 itself guides the light from the light source 200. The main body part 60 is formed of a light transmissive material.
The light from the light source 200 enters the main body part 60 from an end face of a proximal end part of the main body part 60.

Three openings 61 (openings 61a, 61b, 61c) are formed. The shape, the size, the arrangement, and the like of the three openings 61 are similar to those of the openings 41 in the first embodiment. The opening 61a of the three openings 61 is the opening 61 closest to the proximal end part. Note that the number of openings 61 is not limited to three, but may be at least one.

The light transmitting part 62 is formed at one end (distal end part) of the main body part 60. The light transmitting part 62 includes a curved part 62a and a bottom part 62a. The detailed configuration and material of the light transmitting part 62 (the curved part 62a and the bottom part 62a) are the same as or similar to those of the light transmitting part 41 in the first embodiment.

Note that, when the main body part 60 is formed of a material different from a material of the light transmitting part 62, part of the light from the light source 200 may be reflected at an interface between the main body part 60 and the light transmitting part 62, and thus may be lost without entering the light transmitting part 62. Accordingly, the main body part 60 and the light transmitting part 62 are preferably formed of the same material. Further, when the main body part 60 and the light transmitting part 62 are separate members, and are joined to each other to thereby form the medical tube 6, part of the light from the light source 200 may be reflected at a joint face between the main body part 60 and the light transmitting part 62, and thus may be lost without entering the light transmitting part 62. Accordingly, the main body part 60 and the light transmitting part 62 are preferably formed in an integrated manner.

The first reflection layer 63 is provided over an inner circumferential face 60a of the main body part 60 (Fig. 7, Fig. 8). The second reflection layer 64 is provided over an outer circumferential face 60b of the main body part 60 (Fig. 7, Fig. 8). The third reflection layer 65 is provided over a side face of the opening 61 (Fig. 7, Fig. 8).
Although details are described below, with such a configuration, the light having entered the main body part 60 repeats reflections at the first reflection layer 63, the second reflection layer 64, and the third reflection layer 65, to propagate through the inside of the main body part 60.

The first reflection layer 63, the second reflection layer 64, and the third reflection layer 65 are formed of a light-reflective material. The first reflection layer 63, the second reflection layer 64, and the third reflection layer 65 may be formed of different materials, or may be formed of the same material. For example, the first reflection layer 63, the second reflection layer 64, and the third reflection layer 65 may be formed such that the main body part 60 is formed and then coated with a light-reflective material.

### [Propagation of Light]

Next, with reference to Fig. 7, a description is given of propagation of the light from the light source 200 until the light is emitted from the distal end part of the medical tube 6.

When the position of the distal end part of the medical tube 6 is to be detected, first, the light emitted from the light source 200 enters the main body part 60 from the end face of the proximal end part of the main body part 60 (Fig. 7). The light having entered the main body part 60 propagates to the distal end part side of the main body part 60 while repeating reflections at the first reflection layer 63 and the second reflection layer 64 (Fig. 7). The light having arrived at the distal end part of the main body part 60 enters the light transmitting part 62 from the bottom part 62a of the light transmitting part 62 (Fig. 7).

Note that part of the light having entered the main body part 60 arrives at the third reflection layer 65 formed on a side face of the opening 61a farthest from the distal end part, and is reflected thereat (Fig. 6). The light reflected at the third reflection layer 65 propagates through the inside of the main body part 60. Thus, the light having entered the main body part 60 is not emitted from the opening 61 to the outside of the main body part 60.

As described above, the light transmitting part 62 is formed of the same member as that of the light transmitting part 42 in the first embodiment. Accordingly, as in the propagation of light described in the first embodiment, the light having entered the light transmitting part 62 is refracted at the curved part 62a of the light transmitting part 62, and is emitted to various directions.

### Modification Examples

The configuration of the medical tube position detection system 5 has been described above, but the number, the shape, the size, the arrangement, and the like of the openings 61 are not limited to those described above. Modification examples of the opening 61 are described below.

### First Modification Example

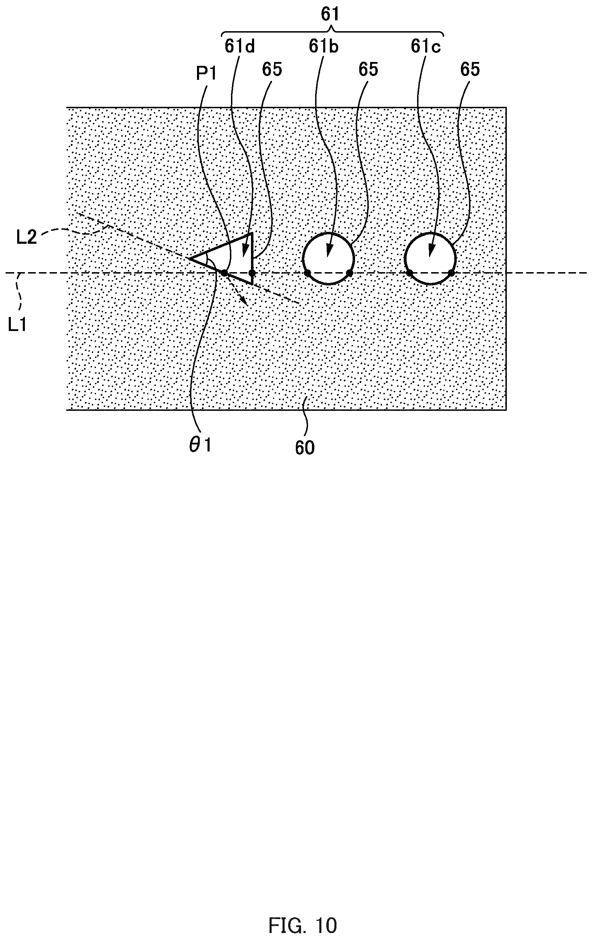

Fig. 9 is a side view of the medical tube 6 in the vicinity of the distal end part thereof in this modification example. Fig. 10 is a developed view of the main body part 60 according to this modification example, and illustrates a view in which the main body part 60 is cut open along one linear line in the longitudinal direction of the main body part 60 and extended to a plane.

In this example, the shape of the opening 61d located at a position farthest from the distal end part, of three openings (the opening 61b, the opening 61c, an opening 61d), is different from the shape of other openings 61. Specifically, the opening 61d has a shape whose width increases toward the distal end part. In this modification example, the opening 61d has a shape of a triangle. Preferably, the triangle is an isosceles triangle, and an apex angle θ1 illustrated in Fig. 10 is smaller than 90 degrees.

A linear line L1 illustrated in Fig. 10 is one of the lines parallel to the longitudinal direction of the main body part 60. The linear line L1 and edges of the openings 61 have six intersection points. Of those six intersection points, the point P1 illustrated in Fig. 10 is an intersection point that is closest to the proximal end part, and is one of the intersection points between the linear line L1 and the edge of the opening 61d. A linear line L2 is a line tangent to the edge of the opening 61d at the intersection point P1.

As illustrated in Fig. 10, for example, the light having entered the main body part 60 and propagating along the linear line L1 arrives at the third reflection layer 65 formed on the side face of the opening 61d, and is reflected at the point P1 (in Fig. 10, the reflected light is indicated by an arrow) . Here, as illustrated in Fig. 10, the light reflected at the third reflection layer 65 propagates to the distal end part side of the main body part 60 (i.e., the direction of propagation is not changed to the proximal end part side of the main body part 60). This is because the apex angle θ1 of the isosceles triangle that is the shape of the opening 61d is smaller than 90 degrees. Accordingly, even after being reflected at the third reflection layer 65, the light from the light source 200 arrives at the distal end part of the main body part 60 more reliably, and easily enters the light transmitting part 62.

### Second Modification Example

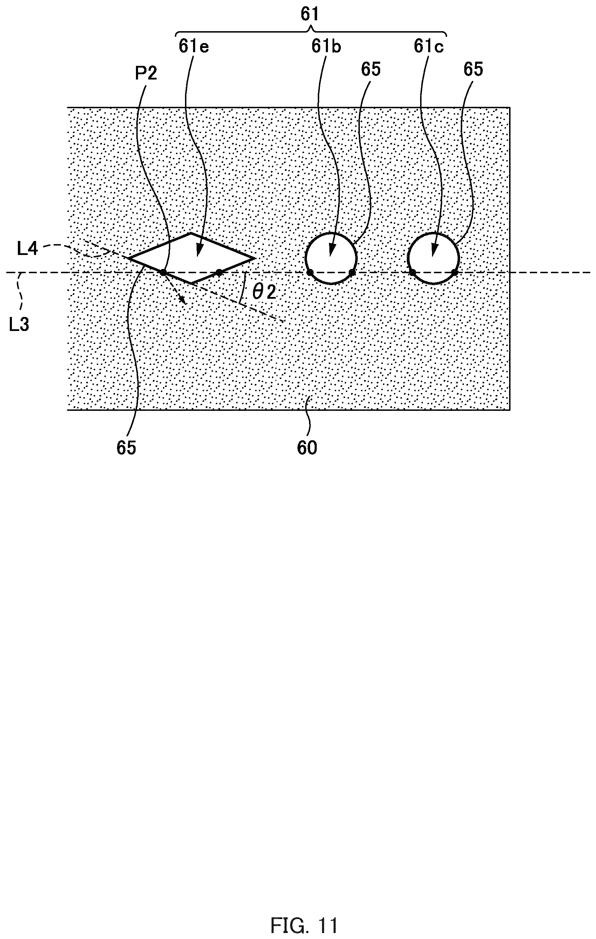

Fig. 11 illustrates a developed view of the main body part 60. This example is different from the first modification example in that the shape of the opening 61e closest to the proximal end part, of three openings 61 (openings 61b, 61c, 61e), is a diamond shape.

A linear line L3 illustrated in Fig. 11 is one of the linear lines parallel to the longitudinal direction of the main body part 60. The linear line L3 and the edges of the openings 61 have six intersection points. Of those six intersection points, a point P2 illustrated in Fig. 11 is an intersection point closest to the proximal end part, and is one of the intersection points between the linear line L3 and the edge of the opening 61e. A linear line L4 illustrated in Fig. 11 is a line tangent to the edge of the opening 61e at the intersection point P2.

In the developed view illustrated in Fig. 11, the light propagating along the linear line L3 is reflected by the third reflection layer 65 at the point P2. Here, the point P2 is located at any position on one of two sides on the proximal end part side at the edge of the opening 61e. In this case, the shape of the opening 61e may be determined such that an angle θ2 formed between the linear line L3 and the linear line L4 is smaller than 45 degrees. With such a configuration, the light reflected at the third reflection layer 65 propagates to the distal end part side.

### Third Modification Example:

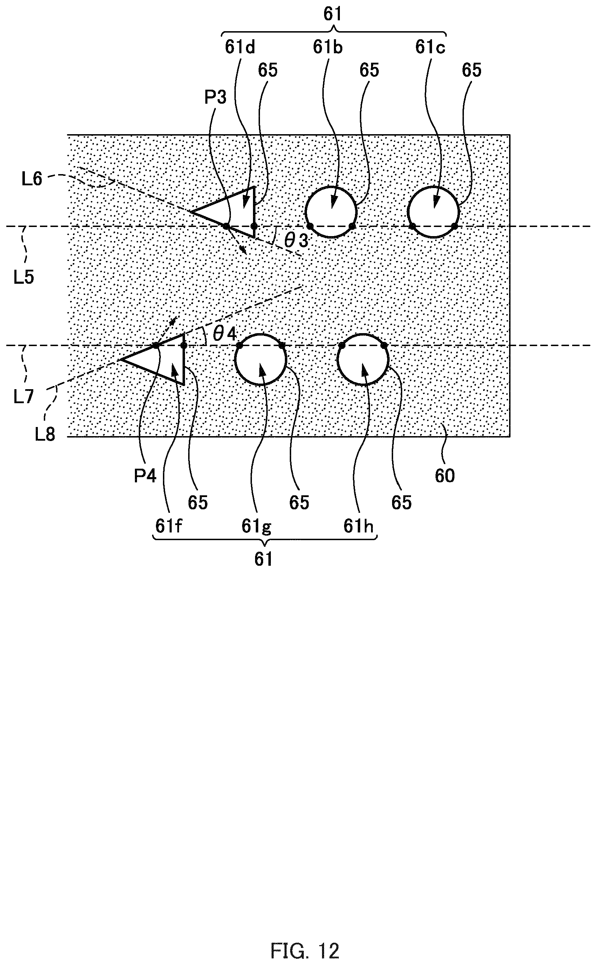

Fig. 12 illustrates a developed view of the main body part 60. This example is different from the first modification example in that six openings 61 (openings 61b, 61c, 61d, 61f, 61g, 61h) are formed.

The six openings 61 are arranged in two rows in the longitudinal direction of the main body part 60. In one row thereof, three openings 61 (the openings 61b, 61c, 61d) are formed as in the first modification example illustrated in Fig. 10. In the other row thereof, three openings 61 (the openings 61f, 61g, 61h) are formed. Of the openings 61f, 61g, and 61h, the opening 61f is the opening 61 closest to the proximal end part. The shape and the size of the openings 61f, 61g, and 61h are the same or similar to the shape and the size of the opening 61d, 61b and 61c, respectively.

A linear line L5 illustrated in Fig. 12 is one of linear lines parallel to the longitudinal direction of the main body part 60. The linear line L5 and the edges of the openings 61 (the openings 61d, 61b, 61c) have six intersection points. A point P3 illustrated in Fig. 11 is an intersection point closest to the proximal end part of those six intersection points, and is one of the intersection points between the linear line L5 and the edge of the opening 61d. A linear line L6 illustrated in Fig. 12 is a line tangent to the edge of the opening 61d at the intersection point P3.

Similarly, a linear line L7 is another one of the linear lines parallel to the longitudinal direction of the main body part 60. The linear line L7 and the edges of the openings 61 (the opening 61f, 61g, 61h) have six intersection points. A point P4 illustrated in Fig. 12 is an intersection point closest to the proximal end part of those six intersection points, and is one of the intersection points between the linear line L7 and the edge of the opening 61f. A linear line L8 illustrated in Fig. 12 is a line tangent to the edge of the opening 61f at the intersection point P4.

In this example, the number, the shape, the size, the arrangement, and the like of the openings 61 may be determined such that both of an angle θ3 formed between the linear line L5 and the linear line L6 and an angle θ4 formed between the linear line L7 and the linear line L8 are smaller than 45 degrees.

### Fourth Modification Example:

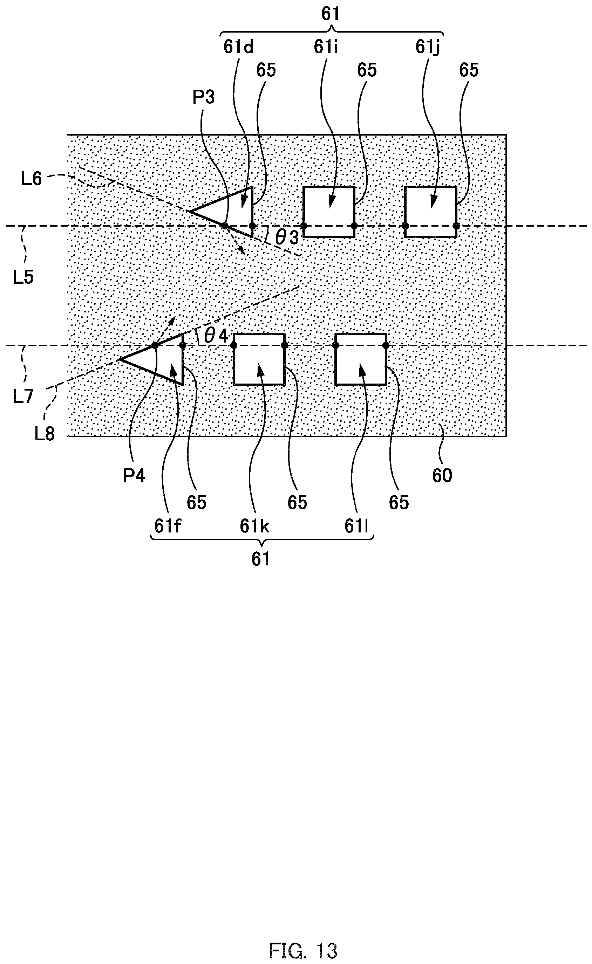

Fig. 13 illustrates a developed view of the main body part 60. In this example, similarly to the example in Fig. 12, six openings 61 (openings 61d, 61f, 61i, 61j, 61k, 61l) are formed.

The six openings 61 are arranged in two rows in the longitudinal direction of the main body part 60. In one row thereof , the three openings 61 (the openings 61d, 61i, 61j) are formed. Of the opening 61d, 61i, and 61j, the opening 61d is the opening 61 closest to the proximal end part. In the other row thereof , the three openings 61 (the openings 61f, 61k, 61l) are formed. Of the openings 61f, 61k, and 61l, the opening 61f is the opening 61 closest to the proximal end part.

This example is different from the example in Fig. 12 in that the openings 61 (the openings 61i, 61j, 61k, 61l) other than the openings 61 (the openings 61d, 61f) closest to the proximal end part, in the openings 61 arranged in each of the rows, have a rectangular shape. Note that the shapes and the sizes of the openings 61i, 61j, 61k, and 61l are not limited to the same shape and the same size, but may be different from one another.

Even with the number, the shape, the size, the arrangement, and the like of the openings 61 as such, the condition described in the second modification example is satisfied. In other words, the openings 61 (the openings 61i, 61j, 61k, 61l) other than the openings 61 (the openings 61d, 61f) closest to the proximal end part may have any shape.

### == Summary ==

As described above, the medical tube **4,** 6 includes the main body part 40, 60 having a tubular shape, at least one opening 41, 61 formed in a side face of the main body part 40, 60, and the light transmitting part 42, 62 that is formed at one end of the main body part 40, 60, the light transmitting part 42, 62 having a hemispherical face shape, the light transmitting part 42, 62 being configured to emit the light from the light source 200.

With the light transmitting part 42, 62 being included, the light from the light source 200 is refracted at a face of the light transmitting part 42, 62, and is emitted. Further, the light transmitting part 42, 62 has a hemispherical face shape, and thus the light from the light source 200 is emitted to various directions. In other words, with such a configuration, the irradiation angle of the light from the light source 200 is larger than in the case without the light transmitting part 42, 62. Accordingly, regardless of the orientation of the distal end part of the medical tube 4, 6 inserted into a body of a patient, the light can be visually recognized from the outside of the body. In other words, the position of the distal end part of the medical tube 4, 6 can easily be detected from the outside of the body.

In the medical tube 4, the main body part 40 allows the light guide 3 to be inserted into the hollow part thereof, the light guide 3 being configured to transmit the light from the light source 200 to the light transmitting part 42. With such a configuration, the light from the light source 200 can efficiently be transmitted to the light transmitting part 42 through the light guide 3.

The medical tube 6 further includes the first reflection layer 63 provided over the inner circumferential face of the main body part 60, the second reflection layer 64 provided over the outer circumferential face of the main body part 60, and the third reflection layer 65 provided over the side face of the at least one opening 61, and the main body part 60 is light transmissive. With such a configuration, the light from the light source 200 propagates through the inside of the main body part 60.
In other words, there is no need to insert the light guide 3, such as an optical fiber or the like, into the hollow part of the main body part 60. Accordingly, reduction in the diameter of the medical tube 6 is facilitated.

In the medical tube 6, the first reflection layer 63, the second reflection layer 64, and the third reflection layer 65 are formed of the same material. With such a configuration, manufacturing of the medical tube 6 is simplified.

In the medical tube 6, the opening 61 located at a position farthest from one end of the medical tube 6 has a shape whose width increases toward the one end. With such a configuration, when the light from the light source 200 is reflected by the third reflection layer 65, the light easily travels to the distal end part side of the main body part 60. Accordingly, the light from the light source 200 can be efficiently emitted from the light transmitting part 62. In other words, it is poassible to reduce loss of the light from the light source 200.

In the medical tube 6, the opening 61 has a shape of a triangle. With such a configuration, after the light from the light source 200 is reflected at the third reflection layer 65, the light travels to the distal end part side of the main body part 40 more easily. Accordingly, the light from the light source 200 can be emitted from the light transmitting part 62 more efficiently. In other words, it is possible to further reduce loss of the light from the light source 200.

In the medical tube 6, the triangle is an isosceles triangle having an apex angle smaller than 90 degrees. Such a configuration facilitates traveling of the light from the light source 200 to the distal end part side of the main body part 60 after being reflected by the third reflection layer 65 more than in the case where the apex angle is 90 degrees or larger. Accordingly, as compared to the case where the apex angle is 90 degrees or larger, the light from the light source 200 can be emitted from the light transmitting part 62 more efficiently. In other words, as compared to the case where the apex angle is 90 degrees or larger, it is possible to further reduce loss of the light from the light source 200.

In the medical tube 4, 6, the main body part 40, 60 and the light transmitting part 42, 62 are formed of one material selected from a group consisting of silicone, polypropylene, elastomer, and a fluorocarbon resin. Such a configuration simplifies manufacturing of the medical tube 4, 6. Further, it is possible to reduce reflection of light at the interface between the main body part 60 and the light transmitting part 62 in the medical tube 6, as compared to the case where the main body part 60 and the light transmitting part 62 are formed of different materials. This makes it possible to efficiently emit the light from the light source 200 from the light transmitting part 42, 62, thereby being able to reduce loss of the light from the light source 200.

The medical tube position detection system 1, 5 includes the light source 200, the main body part 40, 60 having a tubular shape, at least one opening 41, 61 formed in a side face of the main body part 40, 60, the light transmitting part 42, 62 formed at one end of the main body part 40, 60, the light transmitting part 42, 62 having a hemispherical face shape, the light transmitting part 42, 62 being configured to emit the light from the light source 200, and the light guide 3 configured to transmit the light from the light source 200 to the light transmitting part 42, 62, and the light guide 3 being insertable into the main body part 40, 60.

With the medical tube 4, 6 including the light transmitting part 42, 62, the light from the light source 200 is refracted at the face of the light transmitting part 42, 62, and is emitted. Further, with the light transmitting part 42, 62 having a hemispherical face shape, the light from the light source 200 is emitted to various directions. In other words, with such a configuration, the irradiation angle of the light from the light source 200 is larger than in the case without the light transmitting part 42, 62. Accordingly, regardless of the orientation of the distal end part of the medical tube 4, 6 inserted into a body of a patient, the position of the distal end part of the medical tube 4, 6 can easily be detected from the outside of the body.

### [Reference Signs List]

1, 5: Medical tube position detection system;
2: Light source device;
20: Casing;
21: Light collecting part;
200: Light source;
3: Light guide;
4, 6: Medical tube;
40, 60: Main body part;
41, 61: Opening;
42, 62: Light transmitting part;
42A, 62a: Curved part;
42B, 62b: Bottom part;
63: First reflection layer;
64: Second reflection layer;
65: Third reflection layer.

## Claims

1. A medical tube comprising:
a main body part (60) having a tubular shape, the main body part being formed to guide light from a light source (200);
at least one opening (61) formed in a side face of a main body part; and
a light transmitting part (62) formed at one end of the main body part, the light transmitting part having a hemispherical face shape, the light transmitting part being configured to emit the light;
**characterized by**
a first reflection layer (63) provided over an inner circumferential face (60a) of the main body part;
a second reflection layer (64) provided over an outer circumferential face (60b) of the main body part; and
a third reflection layer provided (65) over a side face of the at least one opening, wherein the main body part is light transmissive.

2. The medical tube according to claim 1, wherein the first reflection layer, the second reflection layer, and the third reflection layer are formed of a same material.

3. The medical tube according to claim 1 or 2, wherein the opening located at a position farthest from the one end has a shape whose width increases toward the one end.

4. The medical tube according to claim 3, wherein the opening has a shape of a triangle.

5. The medical tube according to claim 4, wherein the triangle is an isosceles triangle having an apex angle smaller than 90 degrees.

6. The medical tube according to any one of claims 1 to 5, wherein the main body part and the light transmitting part are formed of one material selected from a group consisting of silicone, polypropylene, elastomer, and a fluorocarbon resin.

7. A medical tube position detection system comprising:
a light source (200); and
a medical tube (6) according to any of the previous claims.

## Patentansprüche

1. Ein medizinischer Schlauch, umfassend:
einen Hauptkörperteil (60) mit einer röhrenförmigen Form, wobei der Hauptkörperteil so ausgebildet ist, dass er Licht von einer Lichtquelle (200) leitet;
mindestens eine Öffnung (61), die in einer Seitenfläche eines Hauptkörperteils ausgebildet ist; und
einen lichtleitenden Teil (62), der an einem Ende des Hauptkörperteils ausgebildet ist, wobei der lichtleitende Teil eine halbkugelförmige Flächenform aufweist, wobei der lichtleitende Teil so konfiguriert ist, dass er das Licht emittiert;
**gekennzeichnet durch**
eine erste Reflexionsschicht (63), die über einer inneren Umfangsfläche (60a) des Hauptkörperteils vorgesehen ist;
eine zweite Reflexionsschicht (64), die über einer Außenumfangsfläche (60b) des Hauptkörperteils vorgesehen ist; und
eine dritte Reflexionsschicht (65), die über einer Seitenfläche der mindestens einen Öffnung vorgesehen ist, wobei
der Hauptkörperteil lichtleitend ist.

2. Der medizinischer Schlauch nach Anspruch 1, wobei die erste Reflexionsschicht, die zweite Reflexionsschicht und die dritte Reflexionsschicht aus demselben Material gebildet sind.

3. Der medizinischer Schlauch nach Anspruch 1 oder 2, wobei die Öffnung, die sich an einer Position befindet, die am weitesten von dem einen Ende entfernt ist, eine Form aufweist, deren Breite zu dem einen Ende hinzunimmt.

4. Der medizinischer Schlauch nach Anspruch 3, wobei die Öffnung die Form eines Dreiecks hat.

5. Der medizinischer Schlauch nach Anspruch 4, wobei das Dreieck ein gleichschenkliges Dreieck mit einem Scheitelwinkel von weniger als 90 Grad ist.

6. Der medizinischer Schlauch nach einem der Ansprüche 1 bis 5, wobei der Hauptkörperteil und der lichtleitende Teil aus einem Material gebildet sind, das aus einer Gruppe bestehend aus Silikon, Polypropylen, Elastomer und einem Fluorkohlenstoffharz ausgewählt ist.

7. Ein medizinisches Röhrenpositionserfassungssystem, umfassend:
eine Lichtquelle (200); und
einen medizinischen Schlauch (6) gemäß einem der vorgehenden Ansprüche.

## Revendications

1. Tube médical comportant :
une partie de corps principal (60) ayant une forme tubulaire, la partie de corps principal étant formée pour guider de la lumière provenant d'une source de lumière (200), au moins une ouverture (61) formée dans une face latérale d'une partie de corps principal, et
une partie de transmission de lumière (62) formée à une extrémité de la partie de corps principal, la partie de transmission de lumière ayant une forme de face hémisphérique, la partie de transmission de lumière étant configurée pour émettre la lumière,
**caractérisé par**
une première couche de réflexion (63) disposée au-dessus d'une face circonférentielle intérieure (60a) de la partie de corps principal,
une deuxième couche de réflexion (64) disposée au-dessus d'une face circonférentielle extérieure (60b) de la partie de corps principal, et
une troisième couche de réflexion (65) disposée au-dessus d'une face latérale de la au moins une ouverture, dans lequel la partie de corps principal peut transmettre la lumière.

2. Tube médical selon la revendication 1, dans lequel la première couche de réflexion, la deuxième couche de réflexion et la troisième couche de réflexion sont formées d'un même matériau.

3. Tube médical selon la revendication 1 ou 2, dans lequel l'ouverture située à une position la plus éloignée de l'extrémité a une forme dont la largeur augmente vers l'extrémité.

4. Tube médical selon la revendication 3, dans lequel l'ouverture a une forme de triangle.

5. Tube médical selon la revendication 4, dans lequel le triangle est un triangle isocèle ayant un angle de sommet inférieur à 90 degrés.

6. Tube médical selon l'une quelconque des revendications 1 à 5, dans lequel la partie de corps principal et la partie de transmission de lumière sont formées d'un matériau choisi parmi un groupe constitué de silicone, de polypropylène, d'élastomère et d'une résine fluorocarbonée.

7. Système de détection de position d'un tube médical comportant :
une source de lumière (200), et
un tube médical (6) selon l'une quelconque des revendications précédentes.
